# EUROPEAN PATENT SPECIFICATION

(11) **EP 3 875 600 B1**
(45) Date of publication and mention of the grant of the patent: **18.09.2024**
(21) Application number: 18938497.7
(22) Date of filing: 29.12.2018
(51) Int. Cl.: C12Q 1/68, C12N 5/073, G01N 1/30, G01N 33/569, G01N 33/573

(54) **USE OF HEXOKINASE 2 AS A MARKER OF FETAL TROPHOBLAST CELLS AND IDENTIFICATION METHOD**
VERWENDUNG VON HEXOKINASE 2 ALS MARKER FÜR FÖTALE TROPHOBLASTZELLEN UND IDENTIFIZIERUNGSVERFAHREN
UTILISATION DE HEXOKINASE 2 COMME MARQUEUR DE CELLULES TROPHOBLASTIQUES FOETALES ET PROCÉDÉ D'IDENTIFICATION

(30) Priority: 31.10.2018 CN 201811290842
(43) Date of publication of application: 08.09.2021
(73) Proprietor: Hangzhou Junhui Biotechnology Co., Ltd., Hangzhou, Zhejiang 310000 (CN)
(72) Inventor: SHI, Qihui, Shanghai 200240 (CN); WANG, Chunying, Shanghai 200240 (CN)
(74) Representative: Kuttenkeuler, David
(86) International application number: PCT/CN2018/125798
(87) International publication number: WO 2020/087760

(56) References cited:
- CN-A- 1 798 853
- CN-A- 104 894 229
- CN-A- 107 236 809
- CN-A- 108 410 799
- GUPTA PIYUSHI ET AL: "SIRT6 regulated nucleosomal occupancy affects Hexokinase 2 expression", EXPERIMENTAL CELL RESEARCH, ELSEVIER, AMSTERDAM, NL, vol. 357, no. 1, 4 May 2017 (2017-05-04), pages 98 - 106, XP085074372, ISSN: 0014-4827, DOI: 10.1016/J.YEXCR.2017.05.005
- CRAVEN P A ET AL: "Glucose 6-phosphate-solubilized hexokinase: Some electrophoretic properties", BIOCHIMICA ET BIOPHYSICA ACTA, ELSEVIER, AMSTERDAM, NL, vol. 338, no. 2, 28 February 1974 (1974-02-28), pages 619 - 631, XP023496209, ISSN: 0304-4165, [retrieved on 19740228], DOI: 10.1016/0304-4165(74)90325-0
- SANCHEZ-ALVAREZ R ET AL: "The increase in gap junctional communication decreases the rate of glucose uptake in C6 glioma cells by releasing hexokinase from mitochondria", BRAIN RESEARCH, ELSEVIER, AMSTERDAM, NL, vol. 1039, no. 1-2, 28 March 2005 (2005-03-28), pages 189 - 198, XP027735921, ISSN: 0006-8993, [retrieved on 20050328]
- MAGNANI , M. ET AL : "Hexokinase in human chorionic villi", EARLY HUMAN DEVELOPMENT, vol. 11, no. 2, 1 July 1985 (1985-07-01), pages 149 - 156, XP023125845, ISSN: 0378-3782, DOI: 10.1016/0378-3782(85)90102-1
- BOILEAU, P.: "Hexokinase Isoenzymes in the Rat Placenta", PLACENTA, vol. 19, 31 December 1998 (1998-12-31), pages 439 - 442, XP055702890

## Description

### FIELD OF THE INVENTION

The present invention relates to the technical field of biological cell identification, and in particular to an identification method for fetal trophoblast cells and the use of hexokinase 2 as a biomarker for identifying fetal trophoblast cells.

### BACKGROUND OF THE INVENTION

At present, no passable therapy is available for genetic diseases with chromosomal abnormalities. Therefore, first-trimester detection of abnormalities of fetal genetic information through prenatal diagnosis and active intervention can effectively reduce the rate of birth defects. Currently, prenatal diagnosis methods used in clinical practice are mainly invasive examinations, such as chorionic villus biopsy, amniocentesis, and fetal cord blood testing. However, these invasive examinations cause a risk of infection and miscarriage. Although pre-implantation prenatal diagnosis can be performed on test tube babies, it is only suitable for test tube babies and technically demanding. The non-invasive examinations currently used in clinical practice include B-ultrasound and maternal serum biochemical examinations, but these methods are only auxiliary screening methods of prenatal genetic diseases with limited scope of application rather than prenatal diagnosis methods. Therefore, non-invasive prenatal diagnosis in the first trimester has attracted more and more attention.

The methods reported so far mainly included separating fetal cells and fetal episomal gene fragments from the peripheral blood of pregnant women for detection. Fetal cells in the peripheral blood of pregnant women include fetal nucleated red blood cells and fetal trophoblast cells. However, because the number of fetal cells in the peripheral blood of pregnant women is extremely small and volatile, it is often difficult to isolate a large number of fetal cells for reliable detection. The episomal gene fragments in the peripheral blood of pregnant women have been approved for the detection of triploids of some chromosomes (such as chromosomes 13, 18, 21, etc.). However, the complete genome information of the fetus is unavailable, so the types of genetic diseases that can be detected are very limited, and it is a screening method rather than a diagnostic method.

In 1971, Shettles et al. found fetal trophoblast cells in the cervical mucus of women in the first trimester, which initiated the study of fetal trophoblast cells in the cervix for prenatal diagnosis. The exfoliated trophoblast cells originate from the current pregnancy and are not affected by the previous pregnancy. The trophoblast cells so obtained contain the complete genomic information of the fetus and can be used to detect the chromosomal state of the fetus in the current pregnancy. More importantly, trophoblast cells are relatively rich in cervix uteri, and they can be present as early as in the first trimester, which paves the way for prenatal first-trimester diagnosis. Further studies have shown that trophoblast cells express human leukocyte antigen G (HLA-G), which can be used for the enrichment and separation of fetal trophoblast cells in cervical mucus. Other studies have also reported other markers for identifying fetal trophoblast cells, such as CK7, β-HCG and so on. However, because the cervical exfoliated cells collected in the cervix are complex, consisting of squamous epithelial cells, cervical columnar epithelial cells, cervical glandular epithelial cells, fibroblasts, red blood cells, white blood cells, lymphocytes, and a small amount of trophoblast cells. The markers and detection methods currently available are difficult to accurately detect fetal trophoblast cells in such a complicated cell sample. Therefore, there is a need to identify more sensitive and accurate markers and to develop corresponding detection methods and kits.

### SUMMARY OF THE INVENTION

The invention provides an identification method for fetal trophoblast cells and the use of hexokinase 2 as a biomarker for identifying fetal trophoblast cells, which solves the problems of poor sensitivity and low accuracy in identification of fetal trophoblast cells.

In order to solve the above technical problems, the present invention provides the use of hexokinase 2 (HK2) in the identification or isolation of fetal trophoblast cells, wherein HK2 is used as a biomarker of fetal trophoblast cells.

The use of hexokinase 2 according to the present invention is defined in claim 1.

Optionally, the fetal trophoblast cells are fetal trophoblast cells in cervical mucus.

The hypoxic environment in the uterus in early pregnancy was known to facilitate cell proliferation and angiogenesis in the placenta, and cause the expression of glycolysis-related genes in trophoblast cells. During embryonic development, the extra-villous trophoblast cells cast off to the cervical canal and are wrapped in cervical mucus. These cast-off trophoblast cells obtain energy mainly through glycolysis. On the other hand, it is also known that glucose is firstly phosphorylated under the catalysis of hexokinase (HK) after being transported into cells by glucose transporter (GLUT) on the cell membrane surface during glycolysis. There are 4 subtypes of human HK that have been discovered, which are coded by *HK1, HK2, HK3* and *HK4.* HK1 is widely expressed in almost all mammalian tissues. HK2 is usually expressed in insulin-sensitive tissues (such as fat, bone, and myocardium), but it is highly expressed in a variety of tumor tissues and regulates glucose metabolism by binding with ion channels on the outer mitochondrial membrane. HK3 is often expressed at low levels, while the expression of HK4 is restricted to the pancreas and liver.

The invention also provides a method for identifying fetal trophoblast cells, comprising staining a sample containing fetal trophoblast cells with a fluorescently labeled HK2 antibody substance, and then performing fluorescence detection, and HK2 positive cells are identified as target cells.

The method of the present invention is defined in claim 2.

If the value of fluorescence intensity is above a threshold, the result is set positive, and if the value below the threshold, the result negative. The threshold can be determined by normal methods and technical measures in the art. For example, when the background is basically the same, an absolute value can be directly set to determine the threshold, while when the background influence is strong, the average value + quintuple standard deviation is used to determine the threshold.

Optionally, the sample is obtained from cervical mucus or peripheral blood of a pregnant woman. The liquefied cervical mucus or the enriched peripheral blood is prepared into a single-cell suspension, and the single-cell suspension is spread, in the form of single cells, on a microwell array chip or a glass slide, preferably a microwell array chip.

Optionally, the fluorescently labeled HK2 antibody substance is an HK2 antibody, or a combination of HK2 primary antibody and secondary antibody, wherein the HK2 antibody is labeled with fluorescein or other fluorescent substances, and the HK2 secondary antibody in the combination of the HK2 primary antibody and the secondary antibody is labeled with fluorescein or other fluorescent substances.

Optionally, the identification method further includes: staining the sample with a nuclear stain and performing fluorescence detection, wherein the cells positive for HK2 and positive for nuclear staining are identified as target cells. Nuclear staining is used to eliminate the interference of non-cellular substances on the fluorescence detection result.

After the nucleus is stained, cells with nucleus can be visually identified, and clearly distinguished from non-nuclear substance which, however, does not develop color.

Optionally, the identification method further includes: staining the sample with a nuclear stain and observing the morphology of the nucleus, and excluding cells with regular form of round or elliptical nucleus from target cells. Experiments showed that cells with round or elliptical nucleus are not fetal trophoblast cells.

Also provided is a kit for detecting fetal trophoblast cells, which includes a fluorescently labeled HK2 antibody substance.

However, the kit is not part of the present invention.

Optionally, the kit further includes a nuclear stain.

Optionally, the detection kit for fetal trophoblast cells further includes a microwell array chip to disperse the sample in single cells, facilitating subsequent detection and separation

Optionally, the fluorescently labeled HK2 antibody substance is HK2 antibody, or a combination of HK2 primary antibody and secondary antibody, wherein the HK2 antibody is labeled with fluorescein or other fluorescent substances, and the HK2 secondary antibody in the combination of the HK2 primary antibody and the secondary antibody is labeled with fluorescein or other fluorescent substances.

Optionally, the nuclear stain is selected from nuclear fluorescent dyes, preferably 4',6-diamidino-2-phenylindole (DAPI) or Hoechst series dyes.

Optionally, the detection kit for fetal trophoblast cells is used to detect fetal trophoblast cells in a biological sample of a pregnant woman, wherein the biological sample is a cervical mucus sample or a peripheral blood sample.

The invention described herein can be used to sensitively and reliably detect fetal trophoblast cells in cervical mucus samples or blood samples of women in the first trimester, and is especially useful for prenatal diagnosis in the first trimester.

### BRIEF DESCRIPTION OF DRAWINGS

Figure 1 is a flowchart of the method for identifying fetal trophoblast cells of the invention.
Figure 2 is an image of the area numbered 360 of the microwell array chip loaded with the cell suspension of Sample 1 of the embodiment.
Figure 3 is an image of the area numbered 154 of the microwell array chip loaded with the cell suspension of Sample 1 of the embodiment.
Figure 4 is a HK2 fluorescent antibody staining image corresponding to Figure 2.
Figure 5 is a nuclear DAPI staining image corresponding to Figure 2.
Figure 6 is a HK2 fluorescent antibody staining image corresponding to Figure 3.
Figure 7 is a nuclear DAPI staining image corresponding to Figure 3.
Figure 8 is a DAPI staining image of some HK2-positive and DAPI-positive cells in Sample 1 of an embodiment.
Figure 9 is a statistical distribution of HK2 signal values of cells in the area numbered 1-80 in Sample 1 of an embodiment.
Figure 10 is an identification electrophoretogram of Y chromosome of target cells in samples 1-5 of the embodiment.
Figure 11 is a bright field image of some target cells in Sample 1 of the embodiment.

### DETAILED DESCRIPTION OF THE EMBODIMENTS

The marker, the identification method, the detection kit of fetal trophoblast cells and use thereof are described in the following in conjunction with examples. It should be understood that these examples are only used to illustrate the invention and not to limit the scope of the invention.

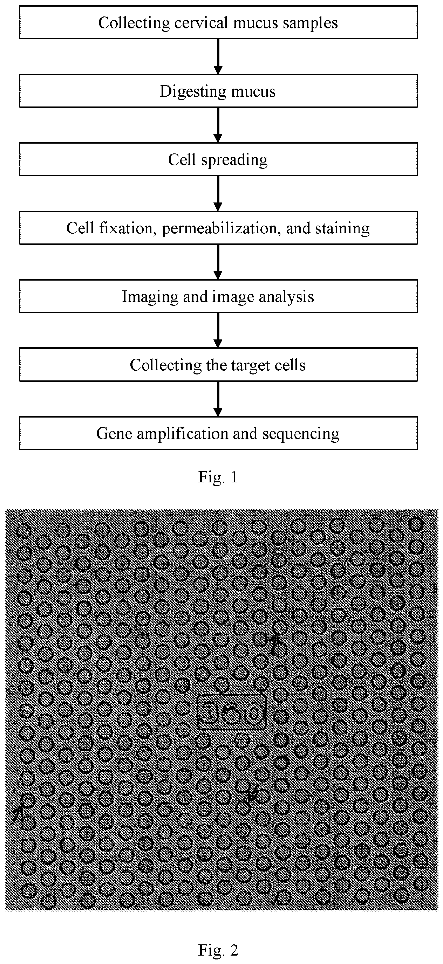

As shown in Figure 1, the method for identifying fetal trophoblast cells mainly includes the following steps:
(a) Collecting cervical mucus samples: using a cell brush to collect cervical mucus from women in the first trimester;
(b) Digesting mucus: cervical mucus is liquefied and prepared into a single-cell suspension.
(c) Cell spreading: spreading the cells in the form of single cells, for example, spreading on a microwell array chip or glass slide;
(d) Cell fixation, permeabilization, and staining: the cells are fixed, permeabilized and stained to identify trophoblast cells derived from the fetus, wherein the reagent used for staining is a certain concentration of fluorescently labeled hexokinase 2 (HK2) antibody substance;
(e) Imaging and image analysis: fluorescence analysis is performed to determine HK2 positive cells with high HK2 expression, which is preliminarily determined as fetal trophoblast cells;
(d) Collecting the target cells: the target cells were obtained through various equipment such as a micromanipulator;
(e) Gene amplification and sequencing: single-cell sequencing or other detection methods known in the art for determining fetal cells is performed to determine whether the cells are fetal-derived trophoblast cells.

The fluorescently labeled HK2 antibody substance is a HK2 antibody directly labeled with fluorescein or other fluorescent substances (such as quantum dots), or a combination of a non-labeled HK2 primary antibody and a secondary antibody labeled with fluorescein or other fluorescent substances.

In an embodiment, the threshold for HK2 positivity is the average value plus quintuple standard deviation of the HK2 signal values of all or part of the cells in the sample.

Further, in order to reduce or eliminate the interference of some non-cellular impurities (e.g. cell debris, bubbles, non-cellular particles, etc.) contained in, or introduced into the sample that adsorb the fluorescent-labeled antibody substance of HK2 with the fluorescence analysis, the embodiment of the method of the invention further includes using the nuclear dye (e.g. nuclear fluorescent dyes, preferably 4',6-diamidino-2-phenylindole (DAPI) or Hoechst series dyes) to stain the sample cells simultaneously with, before or after the HK2 staining. In this case, cells that are positive for both nuclear staining and HK2 staining may be preliminarily identified as fetal trophoblast cells.

In addition to eliminating the interference of non-cellular impurities, the nuclear dye staining described above can also show the morphological characteristics of the cell nucleus to help further improve the accuracy of determining fetal trophoblast cells. The present inventor found that HK2-positive and DAPI-positive cells include cells with different nucleus morphologies, in which most of the cells with irregular (for example, not basically round or elliptical) nucleuses were identified as fetal trophoblast cells by genetic analysis, and the cells with substantially round or irregular nucleuses were usually identified as not fetal trophoblast cells. Single-cell sequencing has verified that the accuracy of the method for detecting fetal trophoblast cells provided by the invention can be as high as 70-85%.

Based on the above detection method, the invention also provides a kit for isolating and identifying fetal trophoblast cells from a cervical mucus sample of a pregnant woman. In an embodiment, the kit includes the above-mentioned hexokinase 2 (HK2) fluorescently labeled antibody substance, a nuclear stain and a microwell array chip. The fluorescently labeled HK2 antibody may be, but is not limited to, FITC labeled HK2 antibody, and the nuclear stain may be 4',6-diamidino-2-phenylindole (DAPI). The microwell array chip includes a plurality of microwells that can accommodate cells and can be addressed to accurately locate and obtain target cells.

Since trophoblast cells are relatively rich in cervical mucus and there are usually no red blood cells, they can generally be detected without the need of enrichment process.

Although the invention is achieved using cervical mucus samples, a skilled person in the art can expect that the method and kit of the invention can also be applied to the detection of fetal trophoblast cells from peripheral blood samples of pregnant women after reading the specification. In the case of peripheral blood, since the number of leukocytes is large while the number of trophoblast cells is very small, the trophoblast cells may optionally be appropriately enriched and then subjected to detection steps such as staining and fluorescence imaging. Enrichment operations such as the removal of red blood cells by red blood cell lysis, density gradient centrifugation, or the removal or partial removal of white blood cells based on CD45 antibody-coated magnetic spheres are known in the art.

The following examples describe in detail the whole process and analysis results of the detection and identification of fetal trophoblast cells using a specific detection kit and the above-mentioned identification method of fetal trophoblast cells to illustrate the effectiveness and superiority of the invention.

### EXAMPLES

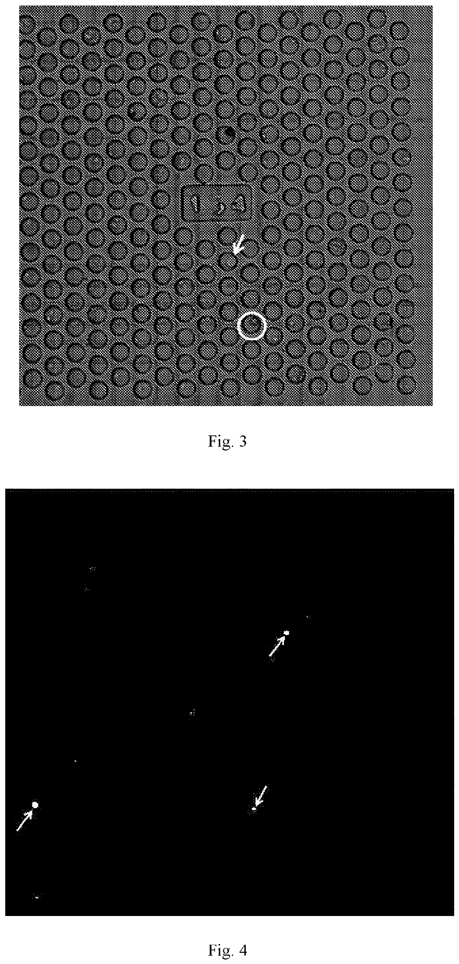

Identification and analysis of fetal cells in cervical mucus for prenatal diagnosis This embodiment includes the following steps:
(1) Using a sterile cell brush to obtain the cervical mucus from 5 women in early pregnancy (6-8 weeks of gestation) (all patients gave informed consent). The brush rotates about 10 times, and the cell brush with the mucus collected by the brush was stored in 2ml Hank's Balanced Salt Solution (HBSS), respectively numbered as Samples 1-5, and placed in a 4-Degree ice box and transported to the laboratory.
(2) The cell brush was taken out and placed in 0.25% pancreatin (containing chelating agent) and incubated at 37° C for 3-5 minutes to digest the mucus, and the cell dispersion was observed under a microscope. Fetal bovine serum (FBS) was used to stop the digestion of pancreatin when cells were sufficiently dispersed.
(3) The 400g suspension obtained in the previous step was centrifuged for 5 minutes and washed with HBSS 3 times. If the cell suspension contains large-sized squamous epithelial cells or impurities, as observed under a microscope, the cell suspension can be filtered with a filter with a pore size of 40-70µm.
(4) The prepared cell suspension was counted and loaded on the microwell array chip at a ratio of 1-1.5 × 10⁵ cells per microwell array chip. In the embodiment, the microwell array chip containing a total of 400 numbered areas, and a total of about 140,000 addressable microwells was used to accommodate and locate cells, wherein the diameter of each micropore was 30 µm, so that the cells were evenly distributed on the chip and sink into the micropores of the chip. As shown in Figures 2 and 3, the images of the two numbered areas of the microwell array chip loaded with the cell suspension of Sample 1 showed that the sample was in a dispersed state of single cells.
(5) 200µl 2% paraformaldehyde (PFA) was added to the chip and the cells were fixed at room temperature for 15 minutes, and then the chip was rinsed 3-5 times with 400 µl of phosphate buffered saline (PBS).
(6) PFA-fixed cells were permeabilized with 200µL 0.5% Triton-100 at room temperature for 15 minutes, and then the chip was rinsed 3-5 times with 400µl of PBS.
(7) The cells after permeabilization are blocked with 3% BSA and Fc receptor blocking agent to block non-specific sites of the cells, and incubated for 1 hour at room temperature.
(8) HK2 fluorescent antibody (e.g. fluorescein FITC-labeled HK2 antibody (1:100 dilution) was added to the chip and the cells were incubated for 1 hour at room temperature, and then the chip was rinsed 3-5 times with 400µl of PBS.
(9) 200µl DAPI was added to the chip and the cells were incubated for 10 minutes at room temperature, and then the chip was rinsed 3-5 times with 400µl of PBS.
(10) The high-content imaging analysis system scanned the chip and analyzed the scan results, wherein the threshold for HK2 positive was the average value plus the quintuple standard deviation of the HK2 signal values of all or part of the cells on the chip. DAPI positive was confirmed when color development is observed.

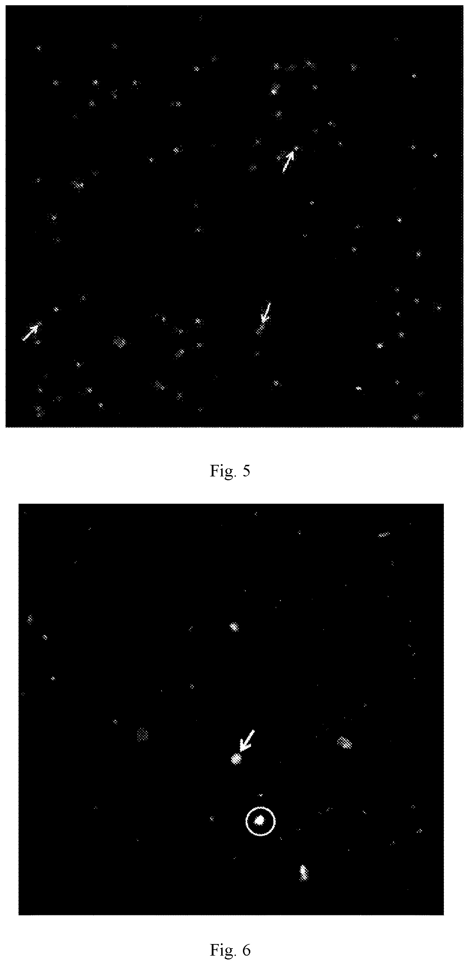

Figure 4 is a HK2 fluorescent antibody staining image of the area numbered 360 corresponding to Figure 2, and the substance in the microwell indicated by the arrow in Figure 4 is HK2 positive. Figure 5 is a nuclear DAPI staining image of the area numbered 360 corresponding to Figure 2, and the substance in the microwell indicated by the arrow in Figure 5 is DAPI positive.

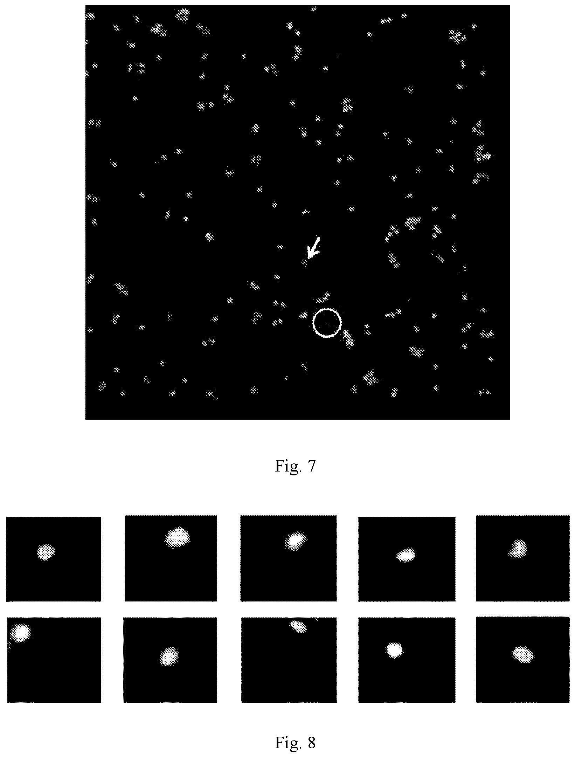

Figure 6 is a HK2 fluorescent antibody staining image of the area numbered 154 corresponding to Figure 3, and the substances in the microwells indicated by the arrow and circle in Figure 6 are both HK2 positive. Figure 7 is a nuclear DAPI staining image of the area numbered 154 corresponding to Figure 3, wherein the substance in the microwell indicated by the arrow has DAPI signal and that indicated by the circle does not have DAPI signal.

It is worth noting that there are very few impurities on the chip that are HK2 positive but DAPI negative. Therefore, the nuclear dye can help us distinguish between cells and non-cellular impurities, so as to eliminate the interference of the very few non-cellular impurities that can adsorb the fluorescently labeled HK2 antibody substance. It can be seen from the staining images of the two chip coding regions shown in Figures 4-7 in which Figures 4 and 5 showed that the cells with high HK2 fluorescence signal (HK2 positive) overlap with DAPI positive cells in the area numbered 360, which indicates that there was no interference caused by the impurities. In the areas shown in Figures 6 and 7, one of the HK2 positive positions did not show DAPI signal, so it was excluded as a cell.

Figure 8 shows the DAPI staining image of some HK2-positive and DAPI-positive cells in Sample 1 of the example, showing different nuclei morphologies, wherein the cell nucleuses of the upper row of cells are irregular and the cells are all verified as fetal trophoblast cells, while the cell nucleuses of the lower row of cells are round or elliptical and none of the cells is verified as fetal trophoblast cells. It shows that the auxiliary identification of cell nuclear morphological characteristics can effectively improve the accuracy of identifying fetal trophoblast cells.

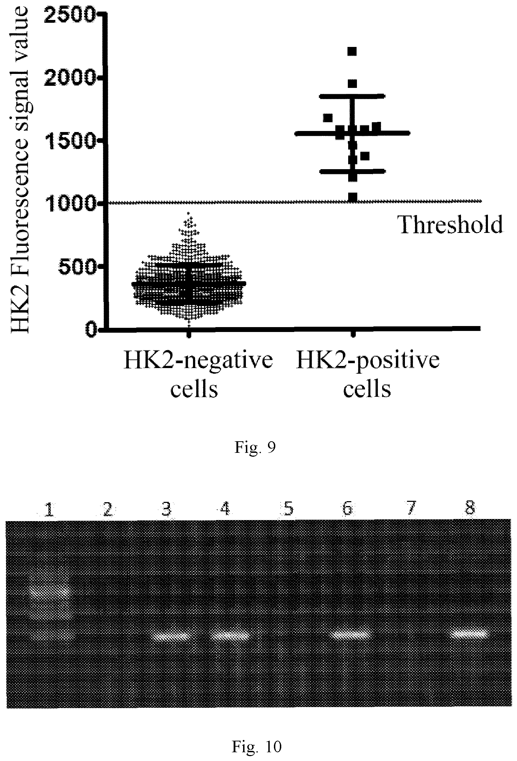

Figure 9 shows a distribution of cells in the area numbered 1-80 in sample 1 obtained by statistical analysis based on the HK2 signal, and the cells with the signal value above the threshold are identified as target cells.

(11) The target cells are selected by the micromanipulation system, and the genome of a single cell is amplified by the MALBAC single cell whole genome amplification kit.

(12) The primers are designed for the sex-determining gene SRY and Y chromosome is tested to determine the fetal origin of the isolated cells.

### Results

Among the 5 samples, the Y chromosome was detected in 3 samples, as shown in the agarose gel electrophoresis image in Figure 10.
Lane 1: DNA marker;
Lane 2: DNA extracted from a cell line derived from women was used as a negative control;
Lane 3: DNA extracted from a cell line derived from men was used as a positive control;
Lane 4: The Y chromosome of the target cell that is HK2-positive/DAPI-positive and meets the morphological characteristics (i.e., irregular nuclear shape) in Sample 1 is detected as positive, indicating that the fetus corresponding to this sample is a male fetus, and the cell can be identified as derived from the fetus; in later clinical testing, the fetus corresponding to this sample was identified as a male fetus, which was consistent with the experimental results.
Lane 5: The Y chromosome of the target cell that is HK2-positive/DAPI-positive and meets the morphological characteristics in Sample 2 is detected as negative, which may be because the fetus corresponding to this sample is a female fetus; in later clinical testing, the fetus corresponding to this sample was identified as a female fetus, which was consistent with the experimental results.
Lane 6: The Y chromosome of the target cell that is HK2-positive/DAPI-positive and meets the morphological characteristics in Sample 3 is detected as positive, indicating that the fetus corresponding to this sample is a male fetus, and the cell can be identified as derived from the fetus; in later clinical testing, the fetus corresponding to this sample was identified as a male fetus, which was consistent with the experimental results.
Lane 7: The Y chromosome of the target cell that is HK2-positive/DAPI-positive and meets the morphological characteristics in Sample 4 is detected as negative, which may be because the fetus corresponding to this sample is a female fetus; in later clinical testing, the fetus corresponding to this sample was identified as a female fetus, which was consistent with the experimental results.
Lane 8: The Y chromosome of the target cell that is HK2-positive/DAPI-positive and meets the morphological characteristics in Sample 5 is detected as positive, indicating that the fetus corresponding to this sample is a male fetus, and the cell can be identified as derived from the fetus; in later clinical testing, the fetus corresponding to this sample was identified as a male fetus, which was consistent with the experimental results.

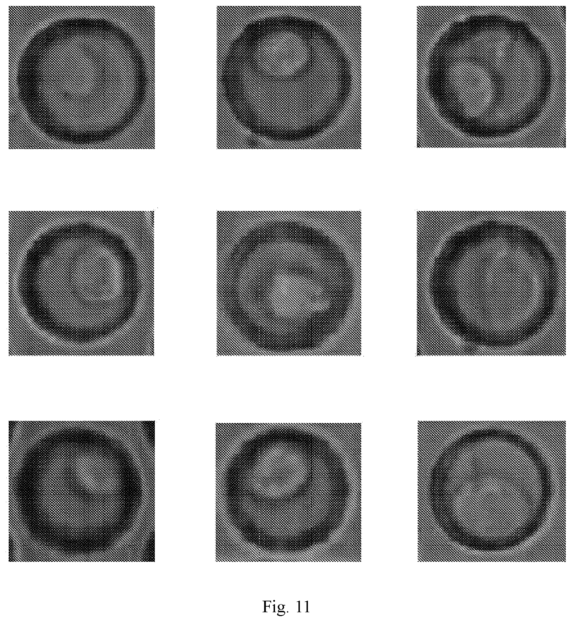

Figure 11 shows a bright field image of some target cells in Sample 1 of the embodiment, wherein the cells were confirmed to be fetal-derived trophoblast cells after Y chromosome detection by single-cell sequencing.

Table 1 shows the statistical data of the number of target cells (HK2 positive, DAPI positive and meeting morphological characteristics) identified in the five samples in the embodiment and the ratio of the number of target cells to the total number of sample cells. It can be found that the proportion of target cells extracted from the sample cells is relatively high, and there are fewer omissions, that is, the sensitivity is high.

**Table 1**

| Sample number | Pregnancy weeks | Total number of sample cells | Total number of target cells | Proportion of target cells |
|---|---|---|---|---|
| 1 | 8 weeks | 4.510 ⁶ | 718 | 1/6267 |
| 2 | 7 weeks | 7×10 ⁵ | 92 | 1/7609 |
| 3 | 6 weeks | 2.4×10 ⁶ | 244 | 1/9836 |
| 4 | 7 weeks | 6.8×10 ⁵ | 112 | 1/6071 |
| 5 | 6 weeks | 2.5×10 ⁵ | 18 | 1/13889 |

For the target cells of Samples 1, 3, and 5, single-cell sequencing can be used to verify the accuracy of the method for determining fetal trophoblast cells according to the invention. The statistical data is shown in Table 2.

**Table 2**

| Sample number | 1 | 2 | 3 |
|---|---|---|---|
| Proportion of fetal trophoblast cells as verified in target cells | 75% | 80% | 75% |

The data in Table 2 shows that the accuracy of the method is also high.

In summary, a relatively large number of target cells in the 5 samples were detected, indicating that the method is of high sensitivity. The single-cell sequencing on the target cells proved that the method has high accuracy at the same time.

It should be understood that, the above-mentioned examples are merely used to illustrate the technical solution of the invention but not to limit the scope of the invention, a skilled person in the art should understand that the technical solutions described in the above-mentioned examples can still be modified, or some or all of the technical features can be equivalently replaced, these modifications or replacements do not cause the essence of the corresponding technical solutions to deviate from the scope of the technical solutions of the examples of the invention.

## Claims

1. Use of hexokinase 2 (HK2) in the identification or isolation of fetal trophoblast cells, wherein the HK2 serves as a biomarker for fetal trophoblast cells.

2. A method for identifying fetal trophoblast cells, comprising staining a sample containing fetal trophoblast cells with a fluorescently labeled HK2 antibody substance, and then performing fluorescence detection, and the HK2-positive cells are identified as target cells.

3. The method of claim 2, wherein the sample is obtained from cervical mucus or peripheral blood of a pregnant woman, and a liquefied cervical mucus or an enriched peripheral blood is prepared into a single-cell suspension, and the single-cell suspension is spread, in the form of single cells, on a microwell array chip or a glass slide, preferably a microwell array chip.

4. The method of claim 2, wherein the fluorescently labeled HK2 antibody substance is an HK2 antibody, or a combination of HK2 primary antibody and secondary antibody, wherein the HK2 antibody is labeled with fluorescein or other fluorescent substances, and the HK2 secondary antibody in the combination of the HK2 primary antibody and the secondary antibody is labeled with fluorescein or other fluorescent substances.

5. The method of claim 2, wherein the method further comprises staining the sample with a nuclear stain and performing fluorescence detection, wherein the cells positive for HK2 and positive for nuclear staining are identified as target cells.

6. The method of claim 2, wherein the method further comprises staining the sample with a nuclear stain and observing the morphology of the nucleus, and excluding cells with a regular form of round or elliptical nucleus from target cells.

## Patentansprüche

1. Verwendung von Hexokinase 2 (HK2) bei der Identifizierung oder Isolierung fötaler Trophoblastzellen, wobei HK2 als Biomarker für fötale Trophoblastzellen dient.

2. Verfahren zur Identifizierung fötaler Trophoblastzellen, umfassend das Einfärben einer Probe, die fötale Trophoblastzellen enthält, mit einer fluoreszenzmarkierten HK2-Antikörpersubstanz und das anschließende Durchführen einer Fluoreszenzdetektion, wobei die HK2-positiven Zellen als Zielzellen identifiziert werden.

3. Verfahren nach Anspruch 2,
wobei die Probe aus Zervixschleim oder peripherem Blut einer schwangeren Frau gewonnen wird, und ein verflüssigter Zervixschleim oder ein angereichertes peripheres Blut zu einer Einzelzellsuspension aufbereitet wird, und die Einzelzellsuspension in Form einzelner Zellen auf einem Mikrotiterplatten-Chip oder einem Objektträger, vorzugsweise einem Mikrotiterplatten-Chip, verteilt wird.

4. Verfahren nach Anspruch 2,
wobei die fluoreszenzmarkierte HK2-Antikörpersubstanz ein HK2-Antikörper oder eine Kombination aus HK2-Primärantikörper und Sekundärantikörper ist, wobei der HK2-Antikörper mit Fluorescein oder anderen fluoreszierenden Substanzen markiert ist und der HK2-Sekundärantikörper in der Kombination aus HK2-Primärantikörper und Sekundärantikörper mit Fluorescein oder anderen fluoreszierenden Substanzen markiert ist.

5. Verfahren nach Anspruch 2,
wobei das Verfahren ferner das Einfärben der Probe mit einem Zellkernfärbemittel und das Durchführen einer Fluoreszenzdetektion umfasst, wobei die für HK2 positiven und für die Zellkernfärbung positiven Zellen als Zielzellen identifiziert werden.

6. Verfahren nach Anspruch 2,
wobei das Verfahren ferner das Einfärben der Probe mit einem Zellkernfärbemittel und das Beobachten der Morphologie des Kerns sowie das Ausschließen von Zellen mit einer regelmäßigen Form eines runden oder elliptischen Kerns aus den Zielzellen umfasst.

## Revendications

1. Utilisation d'hexokinase 2 (HK2) dans l'identification ou l'isolement de cellules du trophoblaste foetal, dans laquelle l'HK2 sert de biomarqueur pour les cellules du trophoblaste foetal.

2. Procédé d'identification de cellules du trophoblaste foetal, comprenant la coloration d'un échantillon contenant des cellules du trophoblaste foetal par une substance anticorps HK2 marquée par fluorescence, puis la détection de la fluorescence, et l'identification des cellules HK2 positives en tant que cellules cibles.

3. Procédé selon la revendication 2,
dans lequel l'échantillon est obtenu à partir de la glaire cervicale ou du sang périphérique d'une femme enceinte, et une glaire cervicale liquéfiée ou un sang périphérique enrichi est préparé en une suspension unicellulaire, et la suspension unicellulaire est étalée, sous forme de cellules individuelles, sur une puce de réseau à micropuits ou une lame de verre, de préférence une puce de réseau à micropuits.

4. Procédé selon la revendication 2,
dans lequel la substance anticorps HK2 marquée par fluorescence est un anticorps HK2 ou une combinaison d'un anticorps primaire HK2 et d'un anticorps secondaire, dans lequel l'anticorps HK2 est marqué par la fluorescéine ou d'autres substances fluorescentes, et l'anticorps secondaire HK2 dans la combinaison de l'anticorps primaire HK2 et de l'anticorps secondaire est marqué par la fluorescéine ou d'autres substances fluorescentes.

5. Procédé selon la revendication 2,
dans lequel le procédé comprend en outre la coloration de l'échantillon avec un colorant nucléaire et la réalisation d'une détection par fluorescence, dans lequel les cellules positives pour HK2 et positives pour la coloration nucléaire sont identifiées en tant que cellules cibles.

6. Procédé selon la revendication 2,
dans lequel le procédé comprend en outre la coloration de l'échantillon avec un colorant nucléaire et l'observation de la morphologie du noyau, et l'exclusion des cellules cibles des cellules ayant une forme régulière de noyau rond ou elliptique.
